# EUROPEAN PATENT APPLICATION

(11) **EP 2 554 197 A1**
(43) Date of publication of application: **06.02.2013**
(21) Application number: 11762171.4
(22) Date of filing: 16.03.2011
(51) Int. Cl.: A61M 5/145

(54) **SYRINGE PUMP**

(30) Priority: 30.03.2010 JP 2010078610
(71) Applicant: Terumo Kabushiki Kaisha, Tokyo 151-0072 (JP)
(72) Inventor: HACHIMURA, Shun, Fujinomiya-shi Shizuoka 418-0015 (JP); KURIMOTO, Masuya, Fujinomiya-shi Shizuoka 418-0015 (JP)
(74) Representative: Uchida, Kenji
(86) International application number: PCT/JP2011/001549
(87) International publication number: WO 2011/121918

(57) **Abstract**

The object is to implement a stable linear operation at a low cost in a slider feed mechanism included in a syringe pump. A syringe pump according to this invention includes a slider assembly and a slider feed mechanism, and feeds a drug filling a syringe. The slider feed mechanism includes a half nut (501), an unmeshing member (510) that pivots in accordance with the operation of a clutch lever, and makes the half nut (501) pivot from a meshed position to an unmeshed position, and a support member (520) that restricts the movement of the half nut (501) in the direction of the rotation axis of the feed screw (303) when the half nut 501 is at the meshed position, and moves the half nut (501) from a support position to a retreat position when the unmeshing member (510) pivots.

## Description

### TECHNICAL FIELD

The present invention relates to a syringe pump.

### BACKGROUND ART

Conventionally, a syringe pump is used for alimentation, blood transfusion, or injection of a drug such as a chemotherapeutant or anesthetic to a patient. The syringe pump is a device in which a syringe filled with a content such as a drug is set on a supporter. After making a slider assembly abut against a cylinder plunger, the user presses an operation start button. The slider assembly thus presses the syringe plunger to feed the content in the syringe. According to this syringe pump, the flow rate can accurately be controlled for a long time.

In such a syringe pump, when the slider assembly presses the syringe plunger, the rotational driving of a motor is converted into a linear operation using a power transmission mechanism called a slider feed mechanism.

More specifically, a half nut is meshed with a feed screw that rotates as the motor is rotationally driven. The half nut is linearly operated in accordance with the rotation of the feed screw, thereby causing the slider assembly connected via a pipe shaft to linearly operate (for example, PTL1).

In addition, a clutch lever is arranged on the slider assembly. When the clutch lever is operated, an inner clutch shaft arranged in the pipe shaft pivots to release the meshed state between the half nut and the feed screw.

### CITATION LIST

### PATENT LITERATURE

PTL1: Japanese Patent Laid-Open No. 2007-306991

### SUMMARY OF INVENTION

### TECHNICAL PROBLEM

In a syringe pump including the above-described slider feed mechanism, generally, a feed screw formed by cutting a metal material is used. The feed screw formed by cutting a metal material is advantageous because the thread portion can have a sawtooth shape (the tooth flank in the liquid feed direction has an angle of 90°), and the meshed half nut hardly disengages. On the other hand, it is also disadvantageous because of high manufacturing cost.

To implement an inexpensive slider feed mechanism, the feed screw may be implemented by, for example, component rolling. However, when the thread face is formed by component rolling, a predetermined restriction is imposed on the workable sectional shape. More specifically, it is impossible to work the flank of the teeth of the feed screw in the liquid feed direction to 90°. For this reason, the half nut readily disengages as compared to the conventional slider feed mechanism. At the time of clutch disengage (a phenomenon that the half nut disengages from the feed screw disengage) or half clutch (a state in which the half nut slightly disengages from the feed screw), stress concentration acts on the distal end of the screw of the half nut, and the tooth break.

The present invention has been made in consideration of the above-described problem, and has as its object to implement a stable linear operation in a slider feed mechanism included in a syringe pump at a low cost.

### SOLUTION TO PROBLEM

In order to achieve the above-described object, a syringe pump according to the present invention has the following arrangement. That is, a syringe pump which includes a slider assembly that abuts against a syringe plunger, and a slider feed mechanism that is connected to the slider assembly and slides the slider assembly in a press direction of the syringe plunger, the syringe pump causing the slider assembly to press the syringe plunger to feed a drug filling a syringe, characterized in that the slider feed mechanism comprises: a half nut that meshes with a feed screw and operates in the press direction of the syringe plunger in accordance with rotation of the feed screw; an unmeshing member that pivots in accordance with an operation of a lever arranged in the slider assembly, and makes the half nut pivot from a meshed position where the half nut meshes with the feed screw to an unmeshed position where the half nut does not mesh with the feed screw; and a support member that supports the half nut in a direction of a rotation axis of the feed screw to restrict movement of the half nut in the direction of the rotation axis of the feed screw when the half nut is at the meshed position, and moves the half nut from a support position to support the half nut in the direction of the rotation axis of the feed screw to a retreat position not to support the half nut when the unmeshing member pivots.

### ADVANTAGEOUS EFFECT OF INVENTION

According to the present invention, it is possible to implement reliable meshing and a stable linear operation in a slider feed mechanism included in a syringe pump at a low cost.

Other features and advantages of the present invention will be apparent from the following descriptions taken in conjunction with the accompanying drawings, in which like reference characters designate the same or similar parts throughout the figures thereof.

### BRIEF DESCRIPTION OF DRAWINGS

The accompanying drawings, which are incorporated in and constitute a part of the specification, illustrate embodiments of the invention and, together with the description, serve to explain the principles of the invention.
Fig. 1 is a view showing an example of the perspective view of a syringe pump according to an embodiment of the present invention;
Fig. 2 is a view showing the arrangement of a slider assembly of the syringe pump;
Fig. 3 is a view showing the arrangement of a slider feed mechanism of the syringe pump;
Fig. 4 is a view showing the perspective view of a nut holder of the slider feed mechanism;
Fig. 5 is a view showing the internal arrangement of the nut holder;
Fig. 6 is a view for explaining the meshed state and the unmeshed state of the nut holder; and
Fig. 7 is a view for explaining the meshed state and the unmeshed state of the nut holder.

### DESCRIPTION OF EMBODIMENTS

The embodiments of the present invention will now be described in detail with reference to the accompanying drawings.

### [First Embodiment]

### <1. Perspective View of Syringe Pump>

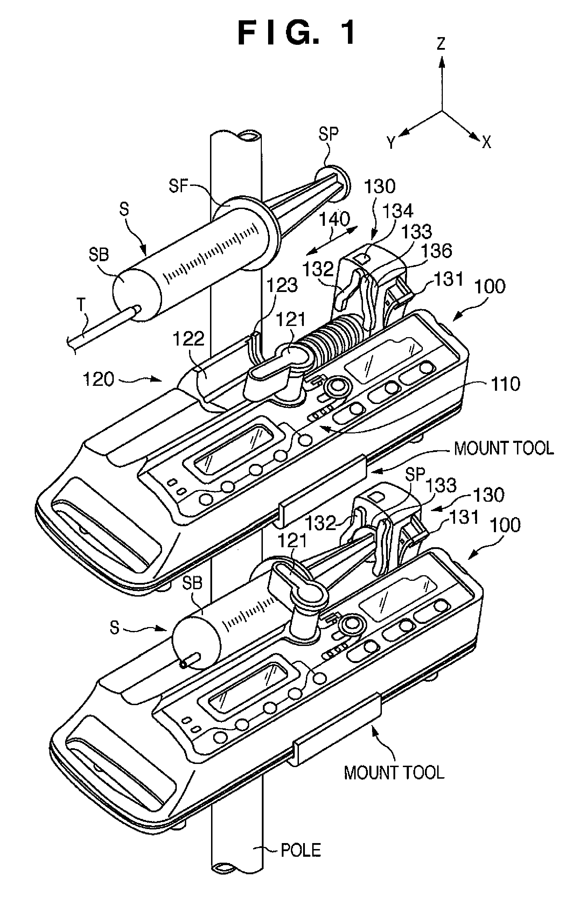

Fig. 1 illustrates the perspective view of a syringe pump 100 according to an embodiment of the present invention. Referring to Fig. 1, the syringe pump attached to the mount tool on the upper side of the pole shows the state before a syringe S is set. The syringe pump attached to the mount tool on the lower side of the pole shows the state after the syringe S is set.

As shown in Fig. 1, the syringe pump 100 includes a syringe fixing portion 120 that makes a flange portion SF to fit while clamping a main body portion SB of the syringe S, thereby attaching the syringe S to the syringe pump 100, a slider assembly 130 connected to a slider feed mechanism (not shown) to press a syringe plunger SP in the direction of an arrow 140 (press direction), and an operation panel 110 used to input various kinds of settings and operation instructions to the syringe pump 100 and display set contents and operation states.

The syringe fixing portion 120 includes a supporter 122 that supports the outer surface of the main body portion SB of the syringe S from the lower side, and a clamp 121 that clamps the outer surface of the main body portion SB of the syringe S with respect to the supporter 122. The X- and Z-axis direction positions of the syringe S on the syringe pump 100 are thus defined. The syringe fixing portion 120 also includes a concave portion 123 that is fitted on the flange portion SF to define the Y-axis direction position of the syringe S.

The slider assembly 130 includes an abutting portion 136 configured to be slidable in the direction of the arrow 140. The abutting portion 136 is connected to the slider feed mechanism (not shown) via a pipe shaft and an inner clutch shaft (neither are shown). When the slider feed mechanism operates, the abutting portion 136 slides in the direction of the arrow 140.

The abutting portion 136 also includes a clutch lever 131. When the clutch lever 131 is pressed, left and right hooks 132 and 133 operate in the opening direction. Simultaneously, the inner clutch shaft pivots, and the meshed state between a feed screw included in the slider feed mechanism and a half nut that meshes with the feed screw is released. Note that the left and right hooks 132 and 133 are biased in the closing direction, and return in the closing direction when the press force on the clutch lever 131 is released.

For this reason, when a user such as a nurse attaches the syringe S to the syringe fixing portion 120, makes the abutting portion 136 abut against the syringe plunger SP while keeping the clutch lever 131 pressed (that is, in the open state of the left and right hooks 132 and 133), and moves the hand off the clutch lever 131, the abutting portion 136 is connected to the syringe plunger SP (see the syringe pump 100 placed on the mount tool on the lower side of Fig. 1). Setting of the syringe S on the syringe pump 100 is thus completed.

The upper surface of the abutting portion 136 is provided with a lamp 134 that lights up or blinks upon detecting that the abutting portion 136 is not correctly connected to the syringe plunger SP. This allows the user to recognize that the abutting portion 136 is not correctly connected to the syringe plunger SP.

On the operation panel 110, devices for receiving various kinds of settings and operation instructions or displaying set contents and operation states are arrayed on the operation panel 110.

### <2. Arrangement of Slider Assembly>

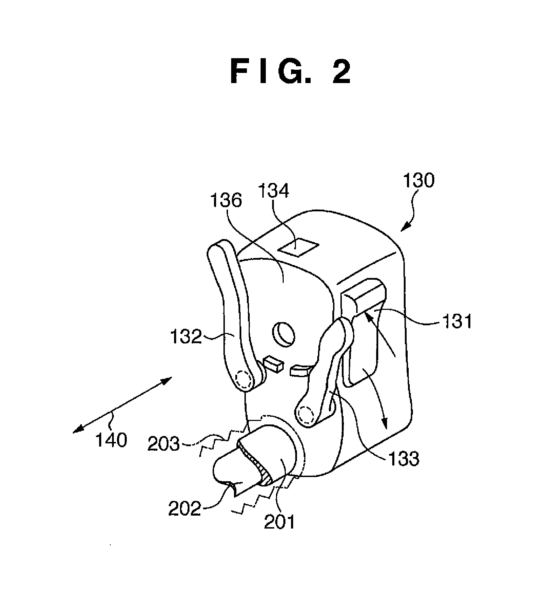

The pipe shaft and the inner clutch shaft connected to the slider assembly 130 will be described next. Fig. 2 is a view showing the outer arrangement of the slider assembly 130.

As shown in Fig. 2, one end of a hollow pipe shaft 201 is connected to the slider assembly 130 in a boot 203 for waterproof. The other end of the pipe shaft 201 is connected to a nut holder (to be described later) arranged in the slider feed mechanism. A half nut incorporated in the nut holder linearly operates as the feed screw rotates. The pipe shaft 201 thus linearly operates, and consequently, the slider assembly 130 slides in the direction of the arrow 140.

An inner clutch shaft 202 is arranged in the pipe shaft 201 and configured to pivot when the clutch lever 131 is pressed. The inner clutch shaft 202 pivots when the clutch lever 131 is pressed. A half nut incorporated in the nut holder thus pivots, and the meshed state to the feed screw is released.

### <3. Arrangement of Slider Feed Mechanism>

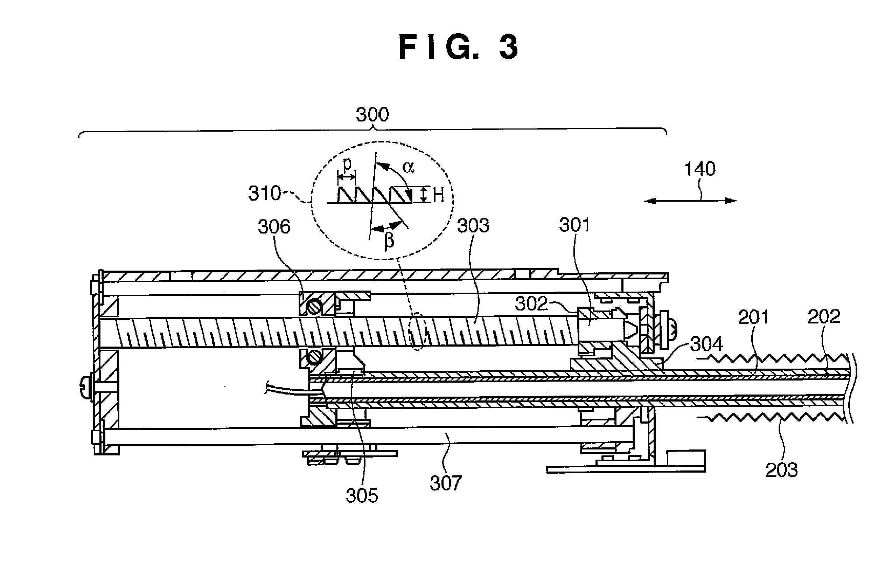

Fig. 3 is a view showing the arrangement of a slider feed mechanism 300 of the syringe pump 100. Referring to Fig. 3, a stepping motor 301 is connected to a feed screw 303 via a gear 302. Guide members 304 and 305 axially support the pipe shaft 201 connected to the slider assembly 130 such that the pipe shaft 201 translates with respect to the feed screw 303.

Note that the feed screw 303 is formed by component rolling of a metal material such as steel. As for the sectional shape of the screw, the flank tilts at an angle α = about 85° with respect to the liquid feed direction (liquid feed direction), and the screw threads have a pitch P = about 0.75 mm, an angle β = about 40°, and a height H = 0.6 mm, as indicated by an enlarged area 310. This arrangement allows to obtain the effect of making the feed screw by easy working at a cost lower than in cutting. In addition, the feed screw can reliably mesh with the half nut, and unmeshing is also easy.

A nut holder 306 includes a half nut that meshes with the feed screw 303. The half nut is configured to pivot as the inner clutch shaft 202 pivots. The half nut pivots between a mesh position where the half nut meshes with the feed screw 303 and an unmeshed position where the meshing is released. One end of the pipe shaft 201 is connected to the nut holder 306. In addition, a parallel shaft member 307 for defining the operation direction is inserted.

With this arrangement, at the meshed position of the half nut, when the feed screw 303 rotates, the half nut linearly operates in the direction of the arrow 140. Accordingly, the nut holder 306 that holds the half nut and the pipe shaft 201 connected to the nut holder 306 slide in the direction of the arrow 140.

On the other hand, when the clutch lever 131 is operated, and the half nut is at the unmeshed position, the user moves the slider assembly 130 in the direction of the arrow 140, thereby moving the nut holder 306 in the direction of the arrow 140. When the user moves the hand off the clutch lever 131 at an arbitrary position, the inner clutch shaft 202 pivots, and the half nut is set in the meshed state again. That is, the user can mesh the half nut with the feed screw 303 at an arbitrary position.

### <4. Arrangement of Nut Holder>

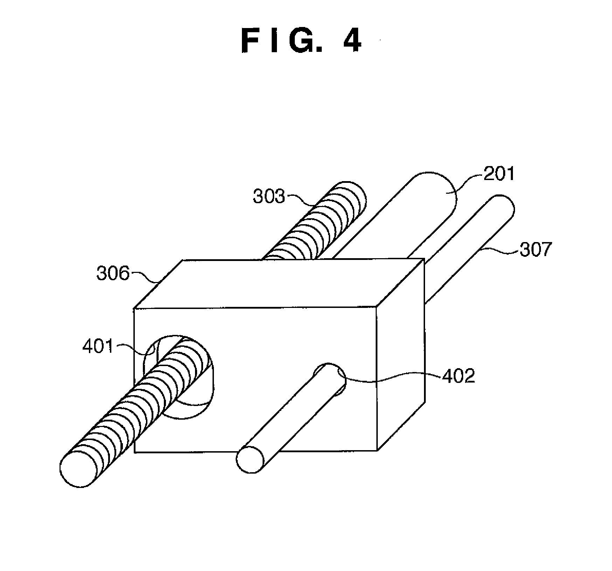

The arrangement of the nut holder 306 will be described next. Fig. 4 is a view showing the perspective view of the nut holder 306.

As shown in Fig. 4, the nut holder 306 has a through hole 401 to receive the feed screw 303, and a sliding hole 402 to slide the parallel shaft member 307 that moves the nut holder 306 in parallel to the feed screw 303 when the incorporated half nut (molded resin product) moves along the feed screw 303 in accordance with rotation of the feed screw 303. Note that the pipe shaft 201 is connected to the housing of the nut holder 306, and the inner clutch shaft 202 arranged in the pipe shaft 201 holds the half nut in the nut holder 306 via a hole (not shown).

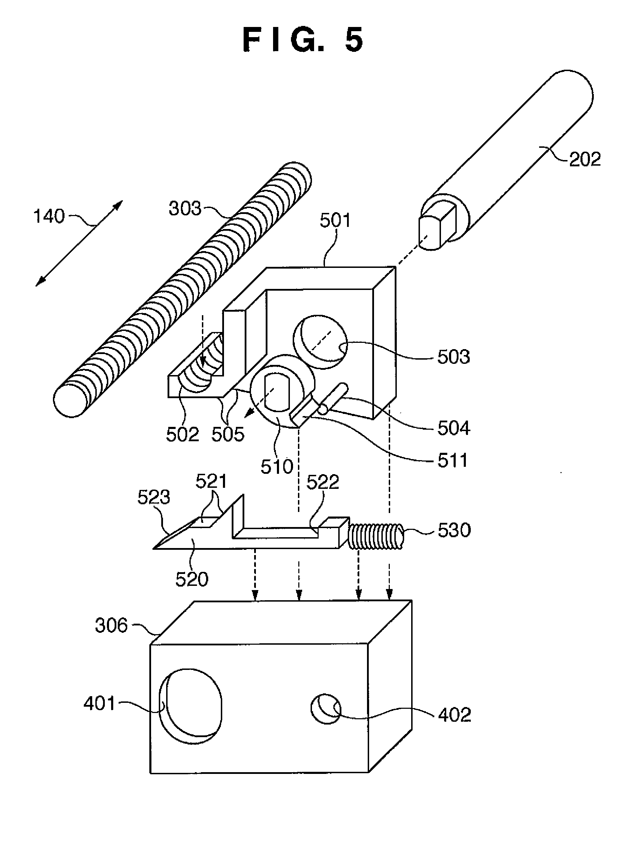

Fig. 5 is a view showing the internal arrangement of the nut holder 306. As shown in Fig. 5, the nut holder 306 incorporates a half nut 501. The half nut 501 is configured to move in the direction of the arrow 140 in accordance with rotation of the feed screw 303 when a thread portion 502 meshes with the feed screw 303.

The half nut 501 has a pivot hole 503 to pivotally hold the half nut 501 with respect to the outer surface of the inner clutch shaft 202 inserted into the pipe shaft 201. When the inner clutch shaft 202 is inserted into the pivot hole 503, the half nut 501 is pivotally held by the inner clutch shaft 202.

The half nut 501 also includes a pivot shaft 504 that is pressed by an unmeshing member 510 to be described later to make the half nut 501 pivot up to the unmeshed position along the outer surface of the inner clutch shaft 202. The half nut 501 further includes a press surface 505 to be pressed by a support member 520 to be described later in the direction of the rotation axis of the feed screw 303 when the half nut 501 is at the meshed position.

The nut holder 306 also incorporates the unmeshing member 510. The unmeshing member 510 is connected to the inner clutch shaft 202 via the pivot hole 503 of the half nut 501 and pivots as the inner clutch shaft 202 pivots. When the unmeshing member 510 pivots, a butt surface 511 of the unmeshing member 510 abuts against the pivot shaft 504 and presses the pivot shaft 504 so that the half nut 501 pivots up to the unmeshed position.

The nut holder 306 also incorporates the support member 520. The support member 520 includes a first surface 521 that presses the press surface 505 of the half nut 501 in the direction of the rotation axis of the feed screw 303 when the half nut 501 is located at the meshed position and meshes with the feed screw 303, a second surface 522 against which the butt surface 511 of the unmeshing member 510 abuts when the unmeshing member 510 has pivoted, and a third surface 523 that presses the half nut 501, which has pivoted to the unmeshed position, in a direction to return to the meshed position.

The support member 520 is configured to be movable between a support position to support the press surface 505 of the half nut 501 in a state in which the half nut 501 meshes with the feed screw 303 and a retreat position to retreat not to interfere with the half nut 501 when it pivots to the unmeshed position.

The nut holder 306 also incorporates an elastic member 530. The elastic member 530 biases the support member 520 in a direction (that is, in the support position direction) reverse to the retreat position direction in which the support member 520 moves when the unmeshing member 510 pivots, and the butt surface 511 of the unmeshing member 510 abuts against the second surface 522 of the support member 520 (that is, functions as a biasing member).

### <5. About Meshed State and Unmeshed State of Half Nut>

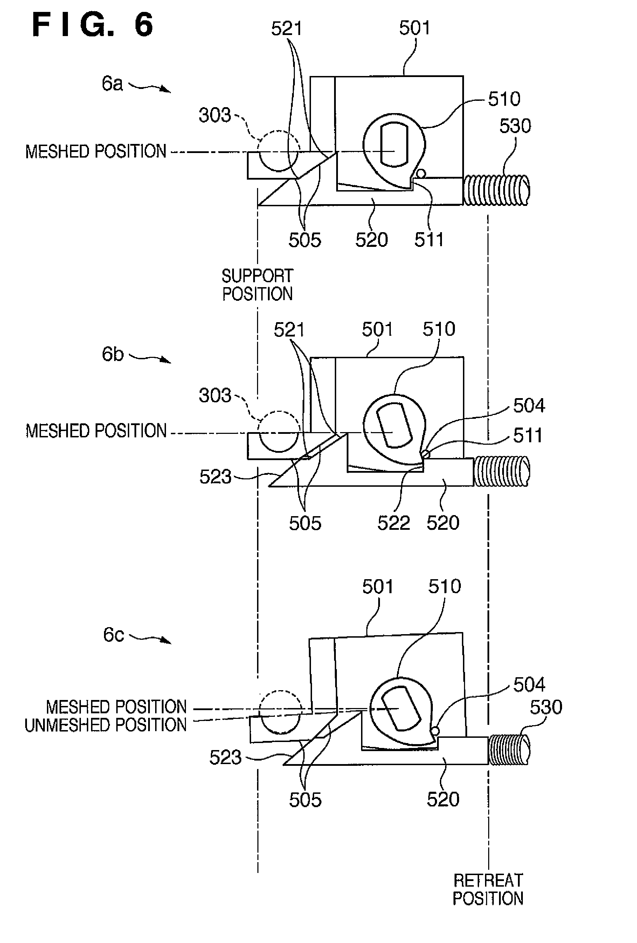

A mechanism for making the half nut 501 pivot between the meshed position and the unmeshed position when the members incorporated in the nut holder 306 operates will be described next with reference to Fig. 6.

In Fig. 6, 6a represents a state in which the half nut 501 meshes with the feed screw 303. In the state in which the half nut 501 meshes with the feed screw 303, the elastic member 530 biases the support member 520 in the support position direction, and the first surface 521 of the support member 520 presses the press surface 505 of the half nut 501 in the direction of the rotation axis of the feed screw 303. The movement of the half nut 501 in the direction of the rotation axis of the feed screw is thus restricted.
For this reason, when the feed screw 303 rotates, the half nut 501 stably linearly operates without disengaging from the feed screw 303.

In Fig. 6, 6b represents a state in which the clutch lever 131 is operated, and the inner clutch shaft 202 pivots. When the inner clutch shaft 202 pivots, the unmeshing member 510 pivots, and the butt surface 511 of the unmeshing member 510 abuts against the second surface 522 of the support member 520, as indicated by 6b of Fig. 6. The support member 520 thus moves in the retreat position direction against the biasing in the support position direction by the elastic member 530.

As a result, the first surface 521 of the support member 520 separates from the press surface 505 of the half nut 501, and a state in which the half nut 501 is not supported by the support member 520 is obtained. When the unmeshing member 510 further pivots in this state, the butt surface 511 of the unmeshing member 510 further abuts against the pivot shaft 504 of the half nut 501.

When the butt surface 511 of the unmeshing member 510 abuts against the pivot shaft 504 of the half nut 501, the half nut 501 pivots in the unmeshed position direction around the inner clutch shaft 202. At this time, since the butt surface 511 of the unmeshing member 510 presses the second surface 522 of the support member 520 as well, the support member 520 further moves in the retreat position direction.

In Fig. 6, 6c represents a state in which the half nut 501 has pivoted up to the unmeshed position. As indicated by 6c of Fig. 6, in the state in which the half nut 501 has pivoted up to the unmeshed position, the unmeshing member 510 presses the half nut 501 via the pivot shaft 504 in the unmeshed position direction, and also presses the support member 520 in the retreat position direction.

Note that in the state shown by 6c of Fig. 6, when the user moves the hand off the clutch lever 131, the support member 520 is moved in the support position direction by the biasing force of the elastic member 530 in the support position direction. The third surface 523 of the support member 520 thus presses the press surface 505 of the half nut 501, and the half nut 501 pivots in the meshed position direction (6b of Fig. 6). When the half nut 501 pivots up to the meshed position, the first surface 521 of the support member 520 completely enters the lower side of the press surface 505 of the half nut 501 and presses the half nut 501 in the direction of the rotation axis of the feed screw 303 (6a of Fig. 6). As described above, in the nut holder 306, the pivot direction of the half nut 501 from the meshed position to the unmeshed position and the moving direction of the support member 520 from the support position to the retreat position are almost perpendicular to each other.

As is apparent from the above description, the syringe pump 100 according to this embodiment can achieve a low cost by forming the feed screw by component rolling and using a half nut formed from a molded resin product.

When the feed screw is formed by component rolling, the half nut readily disengages from the feed screw. In consideration of this, the syringe pump 100 according to this embodiment is configured to cause the support member to support the rear surface of the half nut. This makes it possible to prevent the half nut from disengaging from the feed screw during rotation of the feed screw.

In addition, to make the half nut pivot to the unmeshed position, the support member is configured to be movable between the support position and the retreat position. The operation of moving the support member to the retreat position and the operation of making the half nut pivot to the unmeshed position are implemented by one member (unmeshing member). This allows to make the half nut pivot between the unmeshed position and the meshed position by the same operation as that of the conventional syringe pump.

Furthermore, even if the liquid feed channel is closed by some event during liquid feed, and a load acts on the slider assembly 130, the effect of maintaining the stable meshed state with the feed screw can be obtained because the unmeshing member supports the half nut. Moreover, the unmeshed state can be obtained by an easy operation.

### [Second Embodiment]

In the above-described first embodiment, the pivot shaft is provided to make the half nut pivot, and the butt surface of the unmeshing member abuts against the pivot shaft. However, the present invention is not limited to this. For example, the half nut may be provided with a holding concave portion. An unmeshing member may be arranged in the holding concave portion. When the unmeshing member pivots, the butt surface of the unmeshing member may abut against part of the side wall of the holding concave portion.

In the above-described first embodiment, the butt surface of the unmeshing member abuts against the third surface of the support member, thereby pressing the support member in the retreat position direction. However, the present invention is not limited to this. For example, the support member may be provided with a projecting portion, and the butt surface of the unmeshing member may abut against the projecting portion.

In the above-described first embodiment, the butt surface of the unmeshing member presses the pivot shaft and the third surface of the support member. However, the present invention is not limited to this. The surface against which the butt surface abuts to make the half nut pivot in the unmeshed position direction and the surface against which the butt surface abuts to move the support member in the retreat position direction may separately be provided.

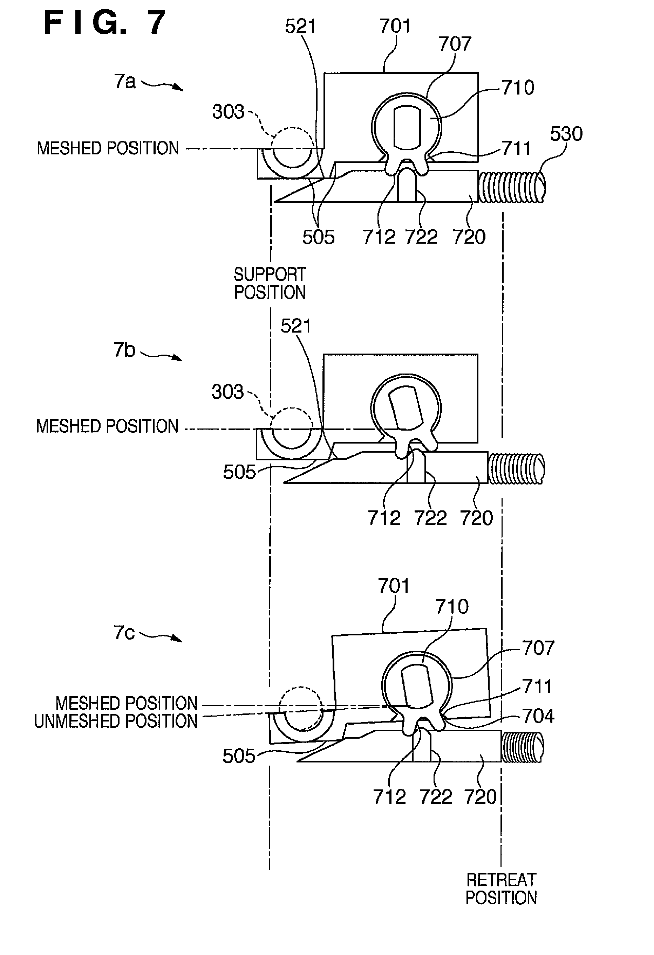

In Fig. 7, 7a to 7c are views showing a mechanism configured such that a half nut 701 is provided with a holding concave portion 707, an unmeshing member 710 is arranged in the holding concave portion 707, and a first butt surface 711 of the unmeshing member 710 abuts against part of the side wall of the holding concave portion, and also, a support member 720 is provided with a projecting portion 722, and a second butt surface 712 different from the first butt surface 711 abuts against the projecting portion 722.

According to the examples of 7a to 7c of Fig 7, when the unmeshing member 710 pivots as an inner clutch shaft 202 pivots, the second butt surface 712 abuts against the projecting portion 722, and the support member 720 moves in the retreat position direction. A first surface 521 of the support member 720 thus separates from a press surface 505 of the half nut 701, and a state in which the half nut 701 is not supported by the support member 720 is obtained (7b of Fig. 7). When the unmeshing member 710 further pivots in this state, the first butt surface 711 of the unmeshing member 710 abuts against part of a side wall 704 of the holding concave portion 707 of the half nut 701.

When the first butt surface 711 of the unmeshing member 710 abuts against part of the side wall 704 of the holding concave portion 707 of the half nut 701, the half nut 701 pivots in the unmeshed position direction around the inner clutch shaft 202. At this time, since the second butt surface 712 of the unmeshing member 710 presses the projecting portion 722 of the support member 720, the support member 720 further moves in the retreat position direction. As a result, the half nut 701 pivots up to the unmeshed position (7c of Fig. 7).

As is apparent from the above description, even when the above-described mechanism is used, the same effect as in the first embodiment can be obtained.

The present invention is not limited to the above embodiments and various changes and modifications can be made within the spirit and scope of the present invention. Therefore, to apprise the public of the scope of the present invention, the following claims are made.

This application claims the benefit of Japanese Patent Application No. 2010-078610, filed March 30, 2010, which is hereby incorporated by reference herein in its entirety.

## Claims

1. A syringe pump which includes a slider assembly that abuts against a syringe plunger, and a slider feed mechanism that is connected to the slider assembly and slides the slider assembly in a press direction of the syringe plunger, the syringe pump causing the slider assembly to press the syringe plunger to feed a drug filling a syringe, **characterized in that**
the slider feed mechanism comprises:
a half nut that meshes with a feed screw and operates in the press direction of the syringe plunger in accordance with rotation of the feed screw;
an unmeshing member that pivots in accordance with an operation of a lever arranged in the slider assembly, and makes said half nut pivot from a meshed position where said half nut meshes with the feed screw to an unmeshed position where said half nut does not mesh with the feed screw; and
a support member that supports said half nut in a direction of a rotation axis of the feed screw to restrict movement of said half nut in the direction of the rotation axis of the feed screw when said half nut is at the meshed position, and moves said half nut from a support position to support said half nut in the direction of the rotation axis of the feed screw to a retreat position not to support said half nut when said unmeshing member pivots.

2. The syringe pump according to claim 1, **characterized by** further comprising a biasing member that biases said support member in a direction from the retreat position to the support position.

3. The syringe pump according to claim 1, **characterized in that**
said half nut is configured to pivot to the unmeshed position when said unmeshing member pivots, and part of said unmeshing member abuts against part of said half nut,
said support member is configured to move to the retreat position when said unmeshing member pivots, and part of said unmeshing member abuts against part of said support member, and
said unmeshing member is configured to abut against part of said support member and then abut against part of said half nut when pivoting in accordance with the operation of the lever.

4. The syringe pump according to claim 1, **characterized in that** a pivot direction of said half nut from the meshed position to the unmeshed position and a moving direction of said support member from the support position to the retreat position are substantially perpendicular to each other.

5. The syringe pump according to claim 1, **characterized in that** the feed screw is formed by component rolling of a metal material and said half nut is formed from a molded resin product.
